# EUROPEAN PATENT APPLICATION

(11) **EP 1 316 618 A2**
(43) Date of publication of application: **04.06.2003**
(21) Application number: 02255738.3
(22) Date of filing: 16.08.2002
(51) Int. Cl.: C12Q 1/68

(54) **Nucleic acids for marking objects**

(30) Priority: 21.11.2001 JP 2001355502
(71) Applicant: ID Technica Corporation, Tokyo (JP); NTT Data Technology Corporation, Tokyo (JP)
(72) Inventor: Itoh, Hisao, c/o ID Technica Corporation, Tokyo (JP); Itakura, Yokio, NTT Data Technology Corporation, Tokyo (JP); Hashiyada, Masaki, Division of Forensic Medicine, Sendai-shi, Miyagi (JP)
(74) Representative: Smith, Norman Ian

(57) **Abstract**

An additive consisting of composition matter made up, according to discrimination information composed of more than one sign which appear repeatedly as more than one pattern, by making each of a number of specific material correspond to each of the patterns, is added to a matter to be discriminated to add discrimination information that there is no fear of being stolen by third party. The more than one signs may be 0 and 1, and the more than one patterns may be 00, 01, 10 and 11. Further, the specific materials may be adenine, cytosine, guanine and thymine, and the composition matter may be artificial DNA. The matter maintaining discrimination information may be such matter to be identified directly such as commodity or ID cards. Or it may be labels to be attached to objects to be identified or matter such as ink printed on objects to be identified.

## Description

This invention relates to an additive which can provide discrimination means for identifying a person in security control, verifying whether a matter or item is a true or valid commodity, and the like, and also relates to matter maintaining discrimination information which contains the additive.

To identify a person or commodity, it is known to use a method where discrimination information is recorded on cards or tags by using magnetism or barcode which are readable in a non-visual way, and to meet the requirement, the discrimination information is read by certain reader such as a barcode reader and verification is conducted.

However, in recent years, as information technology has become more widespread, it is relatively easy to steal information recorded in a usual manner, and particularly the forging of credit cards has become a serious social problem. According to the discrimination information recorded by above-described prior way, to prevent an abuse by a third party, there is the problem that careful attention has to be exercised when using the credit card or other similar item.

Accordingly, the present invention aims to provide an additive to provide discrimination means which reduces the likelihood of theft by a third party, and to provide a matter maintaining discrimination information which contains the additive.

The additive to provide discrimination means, which relates to the present invention, is characterised by comprising a composition matter made up, according to a discrimination information composed of more than one sign repeatedly appearing as more than one pattern, by making each of specific materials be correspond to each of the patterns.

The matter maintaining discrimination information, which relates to the present invention, is characterised by containing the additive.

According to the additive to add discrimination means, by analysing arrangement of material making up a composition matter and by applying the pattern of signs to each of the material, it becomes possible for a person who will conduct an identification to obtain discrimination information. Namely, the composition matter itself of the additive becomes the discrimination means. Here, as it is impossible to obtain the discrimination information without depending on analysing, there is no fear that the information is stolen easily by simple operation such as prior reading operation. Further, as it is impossible to make the discrimination information reappear without making up composition matter by arranging specific materials (ex; the material stated in the claim 4) according to the discrimination information, there is no fear that the information can be formed forged easily. Moreover, as the discrimination means can be added by only adding, despite appearance of commodity needs to be verified, it is possible to form discrimination matter maintaining discrimination information which can be identified.

Further, the matter maintaining discrimination information, which relates to the present invention, is to maintain the discrimination information indirectly through the discrimination means added by the additive. Then, as the discrimination information is that there is no fear of being stolen or forged easily, highly reliable identification can be achieved.

Preferably, said more than one signs are 0 and 1.

In this case, as the discrimination information is digital, it is possible to use the common technology of information processing.

Preferably, said more than one patterns are 00, 01, 10 and 11.

In this case, as digitised discrimination information can be replaced by four kinds of materials, the structure of the composition matter can be relatively simplified.

Preferably, said specific materials are adenine, cytosine, guanine and thymine, and said composition matter is artificial DNA.

In this case, to obtain the discrimination information, the technology of DNA analysis can be adopted.

Artificial DNA means a DNA base array made up by artificially defining the arrangement of four kinds of base, adenine, cytosine, guanine and thymine, that it to say those which make up DNA.

Preferably, said discrimination information is information obtained by encoding combinations of numbers of repeating times of short tandem repeat (STR) in a DNA base array.

In this case, personal discrimination information with high discrimination accuracy, which differs from person to person, can be achieved. Further, by basing the techniques on DNA which every one has, it can be made by standardised method. Furthermore, as combinations of numbers of repeating times of STR is used, and as an encoded process is also conducted, leakage of DNA information itself can be prevented, and then privacy can be protected.

Here, encoded information is to include information processed through a one-way function such as a hash algorithm.

### Brief Description of the Drawings

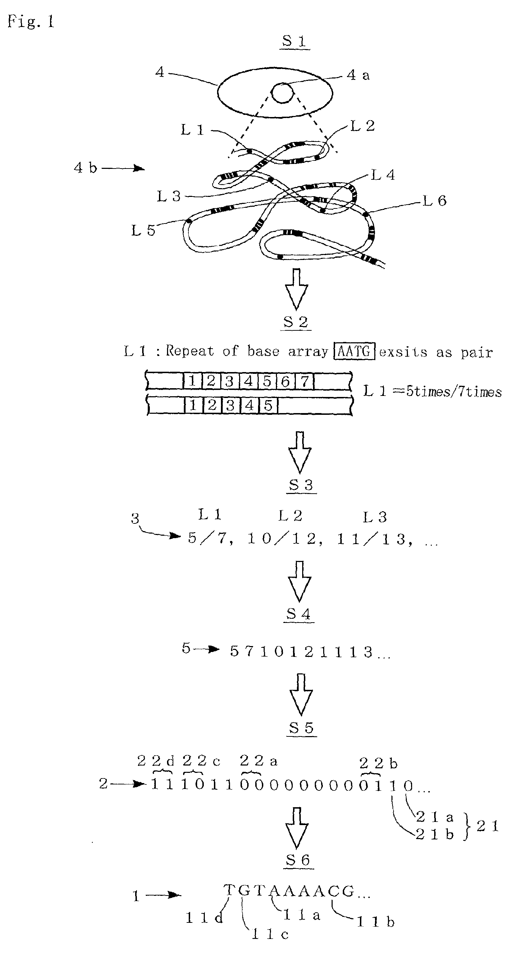

Figure 1 is a flow chart showing an outline of manufacturing process of the additive in respect of an embodiment of the present invention.

### Detailed Description of the Preferred Embodiment

Referring to Figure 1, to be described is an embodiment of additive to add discrimination means in accordance with an embodiment of the present invention. Figure 1 is a flow chart showing an outline of a manufacturing process of the additive in respect of the embodiment.

The additive comprises composition matter 1. The composition matter 1 is made up to provide discrimination information 2 composed or more than one sign 21 repeatedly appearing as more than one patterns 22, by making each of the specific materials 11 correspond to each of the patterns 22.

The additive comprises the composition matter 1, and by analysing arrangement of material 11 making up the composition matter 1 and by applying the patterns 22 of signs to each of the material 11, it is possible for a person who is to conduct an identification to obtain discrimination information 2. Namely, the composition matter 1 itself of the additive becomes the discrimination means. Here, as it is impossible to obtain the discrimination information 2 without depending on analysis, there is no fear that the information is stolen easily by a simply operation such as the prior reading operation. Furthermore, as it is impossible to make the discrimination information 2 reappear without making up the composition matter 2 by arranging specific materials 11 according to the discrimination information 2, there is no fear that the information can be forged easily. Moreover, as the discrimination means can be added by only adding, despite appearance of commodity needs to be verified, it is possible to form discrimination matter maintaining discrimination information which can be identified.

Further, the matter maintaining discrimination information is to maintain the discrimination information 2 indirectly through the discrimination means added by the additive. Then, as the discrimination information 2 is that there is no fear of it being stolen or forged easily, highly reliable discrimination can be achieved. Here, the matter maintaining discrimination information may be such matter to be identified directly such as commodity or ID cards. Or it may be labels to be attached on objects to be identified or such matter like ink printed on objects to be identified. In the case where the matter maintaining discrimination information is ink, there is the advantage that labels to be attached on objects to be identified can be mass-produced by presswork or it can be adapted to any kind of goods where it can be applied.

The plurality of signs 21 comprising the discrimination information are 0 (indicated as 21a in Figure 1) and 1 (indicated as 21b in Figure 1).

By doing this, as the discrimination information 2 is digital, it is possible to use the common technology of information processing.

The plurality of patterns 22 are 00 (indicated as 22a in Figure 1), 01 (indicated as 22b in Figure 1), 10 (indicated 22c in Figure 1) and 11 (indicated as 22d in Figure 1).

By doing this, as digitised discrimination information 2 can be replaced by four kinds of materials, the structure of the composition can be simplified relatively.

The specific materials 11 are adenine 11a, cytosine 11b, guanine 11c and thymine 11d. Thus the composition matter 1 is artificial DNA.

By doing this, to obtain the discrimination information, the technology of DNA analysis can be adopted.

Next to be described is an embodiment of a manufacturing method for the additive to add discrimination means.

The manufacturing method described below is adapted to the case where an additive is manufactured by using a personal DNA base array.

First of all, in first process S1 (process of extracting DNA), DNA 4b is extracted from a cell nucleus 4a in a cell 4 of person to be identified. The cell 4, for example, may be obtained from the mouth of the person to be identified.

Next, in process S2 (process of analysing STR), short tandem repeat (STR) from the extracted DNA 4B is analysed. Base pattern of the analysis is decided for each of STR seat position, and AATG is the base pattern for the example shown in Figure 1. Analysis is conducted at several STR seat positions (locus) L1, L2, L3, L4, L5, L6 ... Figure 1 shows an example where a pattern of AATG appears five times and seven times at STR seat position L1. In Figure 1 of six analysing positions, from STR seat position L1 to L6 are shown; however, the number of analysing positions is not limited and it can be suitably decided according to the discrimination accuracy required for the discrimination information.

The result obtained in the process S2 is, in a process S3 (process of displaying analysing result), displayed as combination 3 of numbers of repeating several times. The combination 3 of numbers of repeating several times shown in Figure 1 is obtained by putting pairs of numbers of repeating times at STR seat places L1, L2, L3 ... in order (the pair is called STR alleles). Here, the numerical values of the STR allele at STR seat positions are put in small order like 5, 7. So are others at other STR seat positions. Then, in process S4 (process of making DNA personal ID), by basing on the combination 3 of numbers of repeating times, DNA personal ID 5 is made.

The DNA personal ID 5 is encoded in process S5 (encoding process), and binary discrimination information 2 is made. The encoding is conducted for the purpose of ethical consideration, or protecting privacy. Therefore, one-way mapping wherein the DNA personal ID 5 is not to be specified from discrimination information 2 is preferable, and a process using a hash algorithm, for example, may be conducted.

Continuously, in a final process S6 (process of converting base array), according to the discrimination information 2 is made in the process S5, material 11 is put so that adenine (11a), cytosine (11b), guanine (11c) and thymine (lid) correspond to pattern 22a (00), to pattern 22b (01), to pattern 22c (10), and to pattern 22d (11) respectively, and the composition matter 1 is made up, and then the additive is produced.

According to the discrimination information 2 produced by this manufacturing method, personal discrimination information with high discrimination accuracy, which differs from person to person, can be achieved. Further, by basing this on DNA which every one has, it can be made by standardized method. Furthermore, as combinations of numbers of repeating times of STR is used, and as an encoded process is also conducted, leakage of DNA information itself can be prevented, and thus privacy can be protected.

### EFFECTS OF THE INVENTION

According to the additive to add discrimination means, which related to the present invention, by analysing an arrangement of material making up a composition matter and by applying the patterns of signs to each of the material, it becomes possible for a person who will conduct identification to obtain discrimination information. Namely, the composition matter itself of the additive becomes the discrimination means. Here, as it is impossible to obtain the discrimination information without depending on analysis, there is no fear that the information is stolen easily by simple operation such as in the prior art reading operation. Further, as it is impossible to make the discrimination information reappear without making up composition matter by arranging specific materials according to the discrimination information, there is no fear that the information can be forged easily. Moreover, as the discrimination means can be added by only adding, despite appearance of commodity needs to be verified, it is possible to form discrimination matter maintaining discrimination information which can be identified.

According to the matter maintaining discrimination information, which relates to the present invention, is to maintain the discrimination information indirectly through the discrimination means added by the additive. Then, as the discrimination information is that there is no fear of being stolen or forged easily, highly reliable discrimination can be achieved.

According to claim 2 and 7, as the discrimination information becomes digital, it is possible to use common information processing technology.

According to claim 3 and 8, as digitized discrimination information can be replaced by four kinds of materials, structure of the composition can be relatively simplified.

According to claim 4 and 9, to obtain a discrimination information, the technology of DNA analysis can be adapted.

According to claim 5, 10, 11 and 12, personal discrimination information with high discrimination accuracy, which differs from each person, can be achieved. Further, as basing on DNA every one has, it can be made by standardized method. Furthermore, as combination of numbers of repeating time of STR is used, and as encoded process is also conducted, leakage of DNA information itself can be prevented, and then privacy can be protected.

## Claims

1. An additive which can provide discrimination means comprising a composition of matter which incorporates discrimination data (2) comprising more than one sign which can appear as repeating patterns (22) each pattern corresponding to a particular or specific material.

2. An additive as claimed in claim 1, wherein said more than one signs (21) are 0 (21a) and 1 (21b).

3. An additive as claimed in claim 1, wherein said more than one patterns (22) are 00 (22a), 01 (22b), 10 (22c) and 11 (22d).

4. An additive as claimed in any one of claims 1 to 3, wherein said specific materials (11) are adenine (11a), cytosine (11b), guanine (11c) and thymine (11d), and said composition matter (1) is artificial DNA.

5. An additive as claimed in any one of claims 1 to 4, wherein said discrimination information (2) is information obtained by encoding a combination (3) of numbers of repeating times of short tandem repeat (STR) in DNA base array.

6. A matter maintaining discrimination information which contains an additive comprising composition matter (1) made up, according to a discrimination information (2) composed of more than one signs (21) appearing repeatedly as more than one pattern (22), by making each of specific materials (11) correspond to each of the patterns (22).

7. A matter maintaining discrimination information as claimed in claim 6, wherein said more than one sign (21) are 0 (21a) and 1 (21b).

8. A matter maintaining discrimination information as claimed in claim 6, wherein said more than one pattern (22) are 00 (22a), 01 (22b), 10 (22c) and 11 (22d).

9. A matter maintaining discrimination information as claimed in any one of claims 6 to 8, wherein said specific material (11) are adenine (11a), cytosine (11b), guanine (11c) and thymine (11d), and said composition matter (1) is artificial DNA.

10. A matter maintaining discrimination information as claimed in any one of claims 6 to 9, wherein said discrimination information (2) is an information obtained by encoding a combination (3) of numbers of repeating times of short tandem repeat (STR) in DNA base array.
